# EUROPÄISCHE PATENTSCHRIFT

(11) **EP 1 961 367 B1**
(45) Veröffentlichungstag und Bekanntmachung des Hinweises auf die Patenterteilung: **04.05.2011**
(21) Anmeldenummer: 08151386.3
(22) Anmeldetag: 13.02.2008
(51) Int. Cl.: A61B 1/00

(54) **Elektrische Steckereinrichtung mit integrierten hydraulischen/pneumatischen Anschlüssen**
Electric plug device with integrated hydraulic/pneumatic connections
Dispositif de prise électrique doté de raccords hydrauliques/pneumatiques intégrés

(30) Priorität: 22.02.2007 DE 102007000109
(43) Veröffentlichungstag der Anmeldung: 27.08.2008
(73) Patentinhaber: Invendo Medical GmbH, 69469 Weinheim (DE)
(72) Erfinder: Schütz, Günter, 86405, Meitingen (DE)
(74) Vertreter: Winter, Brandl, Fürniss, Hübner, Röss, Kaiser, Polte - Partnerschaft

(56) Entgegenhaltungen:
- EP-A- 0 855 299
- DE-A1- 3 624 442
- GB-A- 2 288 079
- US-A- 4 720 128
- US-A- 5 812 356
- US-A1- 2003 162 448
- US-B1- 6 851 427

## Beschreibung

Die vorliegende Erfindung betrifft eine elektrische Steckverbindung mit integrierten hydraulischen/pneumatischen Anschlüssen sowie vorzugsweise mit einer integrierten Steuerungselektronik.

Aus dem Stand der Technik sind Steckereinrichtungen zur Verbindung von elektrischen oder hydraulischen/pneumatischen Geräten bekannt. Elektrische Steckereinrichtungen bestehen in der Regel aus einem elektrisch isolierenden Kunststoffgehäuse sowie einer Anzahl von elektrischen Kontakten, die vorzugsweise in einem von dem Gehäuse umgebenen, auf einer Stirnseite hin offenen Aufnahmeraum geschützt untergebracht sind. Innerhalb des Steckergehäuses sind elektrische Verbindungskabel mit den elektrischen Kontakten verschraubt oder verlötet, wobei das Steckergehäuse derart mit dem Kabel in Eingriff ist, dass bei einer mechanischen Belastung des Kabels die Verlöt- bzw. Verschraubpunkte mit den elektrischen Kontakten möglichst mechanisch unbelastet bleiben.

Des weiteren sind hydraulische/pneumatische Steckereinrichtungen bekannt, mittels denen ein oder eine Mehrzahl von druckbeaufschlagten Schläuchen an Druckverbraucher anschließbar sind. Derartige Steckereinrichtungen weisen in der Regel Anschlagflansche auf, an denen gegebenenfalls zusätzliche Dichtungsringe vorgesehen sind, sowie Schraubmittel, vorzugsweise in Form von Überwurfmuttern, die mit entsprechenden Gewinden auf Seiten des Druckverbrauchers oder der Druckquelle in Wirkeingriff bringbar sind, um einen druckdichten Kontakt zwischen Stecker und Druckverbraucher/Druckquelle herzustellen.

Es hat sich gezeigt, dass derartige allgemein bekannte Steckerverbindungen sich dann als unökonomisch bzw. überhaupt nicht anwendbar erweisen, wenn bspw. eine Vielzahl von hydraulischen/pneumatischen sowie elektrischen Verbindungen an einem Gerät vorgesehen sind und darüber hinaus der vorhandene Anschlussbauraum sehr klein ist.

Aus der EP 0 855 299 A2 ist ein Stecker sowie eine Steckdose bekannt, über die sowohl ein elektrischer wie auch pneumatischer/hydraulischer Kontaktschluss herstellbar ist.

Die Aufgabe der vorliegenden Erfindung darin, eine Steckereinrichtung zu schaffen, mittels der elektrische sowie hydraulische/pneumatische Verbindungen möglichst Platz sparend und ökonomisch herstellbar sind.

Diese Aufgabe wird durch eine Steckereinrichtung mit den Merkmalen des Patentanspruchs 1 gelöst.

Die Erfindung wird nachstehend anhand bevorzugter Ausführungsbeispiele unter Bezugnahme auf die begleitenden Zeichnungen näher erläutert.
Fig. 1 zeigt eine Vorderansicht der Steckdose der erfindungsgemäßen Steckereinrichtung gemäß einem bevorzugten Ausführungsbeispiel der Erfindung,
Fig. 2 zeigt eine teilgeschnittene Draufsicht der Steckdose gemäß Fig. 1,
Fig. 3 zeigt eine Seitenansicht der Steckdose gemäß Fig. 1,
Fig. 4 zeigt eine teilgeschnittene Draufsicht einer Steckdose gemäß der Fig. 1, eingebaut in einem Y-Stück (nicht Teil der Erfindung) eines Endoskops, Katheters oder Trokars,
Fig. 5 zeigt eine Rückansicht eines Steckers der Steckereinrichtung gemäß dem bevorzugten Ausführungsbeispiel der vorliegenden Erfindung,
Fig. 6 zeigt eine Perspektivenansicht des Steckers gemäß Fig. 5 und

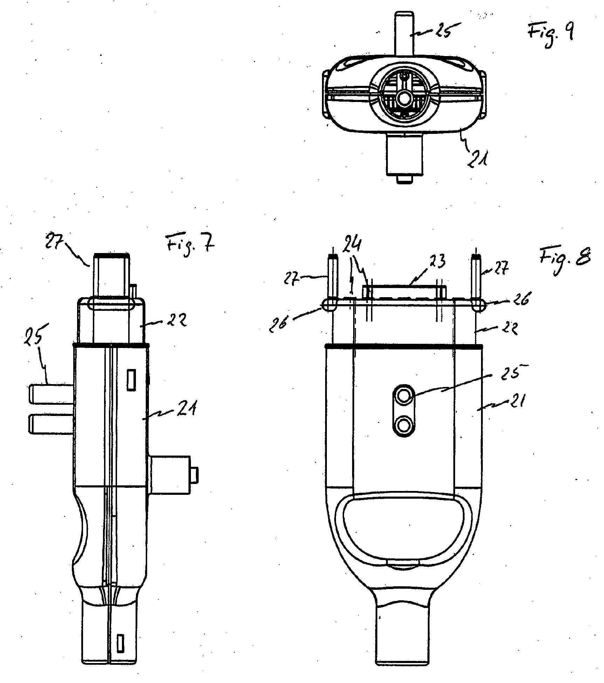

Fig. 7 zeigt eine Seitenansicht des Steckers gemäß Fig. 5 und
Fig. 8 zeigt eine Draufsicht eines Y-Stücks (nicht Teil der Erfindung) mit einer eingebauten Steckdose gemäß Fig. 1,
Fig. 9 zeigt eine Rückansicht eines Y-Stücks (nicht Teil der Erfindung),

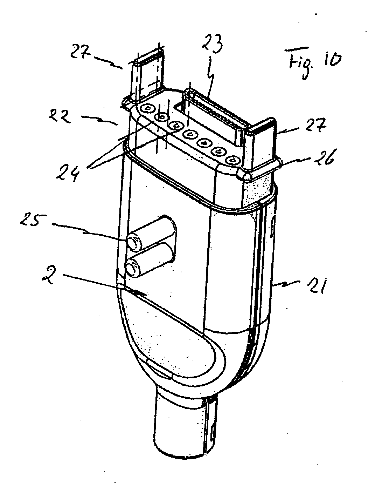

Fig. 10 zeigt eine Perspektivenansicht eines Y-Stücks (nicht Teil der Erfindung)
Fig. 11 zeigt eine prinzipielle Perspektivenansicht eines Y-Stücks.

Die erfindungsgemäße Steckereinrichtung nach einem bevorzugten Ausführungsbeispiel der Erfindung umfasst eine Steckdose 1 sowie einen speziell hierfür angepassten Stecker 2. Zur Veranschaulichung ist ein Y-Stück 3 eines Endoskops, Katheters, Trokars oder dergleichen medizinische Arbeitsgerätschaft mit der Steckdose 1 oder dem Stecker 2 gemäß dem bevorzugten Ausführungsbeispiel der Erfindung ausgerüstet.

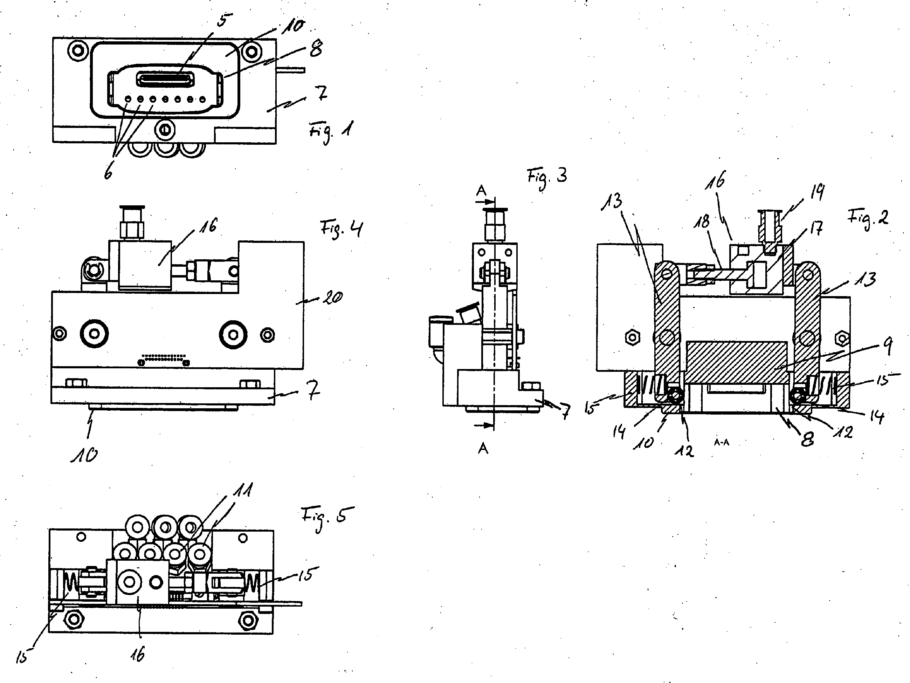

Gemäß der Figuren 1 und 2 handelt es sich bei der erfindungsgemäßen Steckdose 1 um eine so genannten Kombinationssteckdose zur gleichzeitigen Herstellung elektrischer sowie hydraulischer/pneumatischer Verbindungen. Hierfür hat die Steckdose 1 einen Kontaktschacht 4, in welchem eine Anzahl von elektrischen Anschlüssen 5 wie auch hydraulischen und/oder pneumatischen Anschlüssen 6 untergebracht sind. Der Kontaktschacht 4 wird von einem Gehäusestück der Steckdose gebildet, bestehend aus einer Frontplatte 7, an der eine im Wesentlichen rechteckige Öffnung ausgeschnitten ist. In diese Öffnung ist ein Buchsenelement 8 eingeschoben, welches an einer Stirnseite hin offen ist, wohingegen ein der gegenüberliegenden Rückseite eine Verschlussplatte 9 mit darin eingelassenen Kontaktklemmen angeordnet ist. Um die offenen Stirnseite der Buchse 8 herum ist eine Anschlagplatte 10 einstückig daran ausgebildet, welche nach Art eines Flanschs an der Gehäuseplatte 7 anliegt und damit die Buchse 8 fest fixiert.

Gemäß der Fig. 1 sind des weiteren in der Verschluss- oder Rückplatte 9 eine Anzahl von Durchgangsbohrungen ausgebildet, die an der Hinterseite der Rückplatte 9 zu Gewindebohrungen ausgeformt sind. In diese Gewindebohrungen sind hydraulische Anschlusszapfen 11 eingeschraubt, an welche Druckleitungen (nicht gezeigt) fluiddicht (bspw. per Klemmringe) anschließbar sind. Alternativ hierzu können die Anschlusszapfen 11 auch einstückig mit der Rückplatte 9 ausgebildet, oder an diese angeschweißt/angelötet sein. Vorzugsweise werden die Durchgangsbohrungen an der Vorderseite der Rückplatte 9 von ringförmigen Nuten umgeben, in die Dichtungsringe eingeklebt sind (nicht dargestellt) .

Wie insbesondere in der Fig. 2 dargestellt ist, weist die in die Gehäuseplatte 7 eingeschobene Buchse 8 seitliche Öffnungen oder Schlitze 12 auf, welche vorzugsweise eine Rechtecksform haben.

An dem Gehäuse der Steckdose 1 sind beidseits der Buchse 8 mittig drehbar gelagerte Hebel 13 angeordnet, die an ihren einen Hebelenden jeweils eine daran drehbar gelagerte Klemmwalze 14 aufweisen. Des weiteren ist jeder Hebel 13, welcher sich parallel zur Steckrichtung der Buchse 8 an deren Seite erstreckt, mittels eines Federelements 15 derart vorgespannt, dass die beiden sich gegenüberliegenden Klemmwalzen 14 in die seitlichen Öffnungen oder Schlitze 12 der Buchse 8 elastisch eingedrückt werden. Die sich gegenüberliegenden anderen Enden der beiden Klemmhebel 13 sind mit einem Hebel-Betätigungsmechanismus 16 gekoppelt.

In einer bevorzugten Ausführungsform der Erfindung besteht dieser Betätigungsmechanismus 16 aus einem Hydraulik/Pneumatikzylinder 17, in dem ein Kolben mit einer axialen Bewegungsbeschränkung gleitgelagert ist. Der Zylinder 17 ist dabei drehbar an dem einen Klemmhebel-Ende anscharniert, wohingegen der Kolben 18 an dem Ende des gegenüberliegenden Klemmhebels 13 drehbar angelenkt ist. Der Zylinder 17 weist ferner einen Hydraulik/Pneumatikanschluss 19 für dessen Betätigung auf.

Es sei an dieser Stelle darauf hingewiesen, dass an Stelle der vorstehend beispielhaft beschriebenen Zylinder/Kolben-Anordnung auch eine andere Art eines Betätigungsmechanismus vorgesehen werden kann der nicht Teil der vorliegenden Erfindung ist. Beispielsweise ist auch eine elektromotorische Einheit mit nachgeschaltetem Getriebe (Zahnstange, Spindel etc.), ein Piezoelement, eine direkt wirkende Tauchspule oder alternativ ein System aus Spule und axial bewegbarem Anker anwendbar.

Vorzugsweise kann die erfindungsgemäße Steckdose 1 mit einer daran fixierten elektronischen Einrichtung 20 in Form einer Schaltplatine ausgerüstet sein, über die bspw. Steuersignale zur Aktivierung/Deaktivierung der Betätigungseinrichtung 16 gegeben werden.

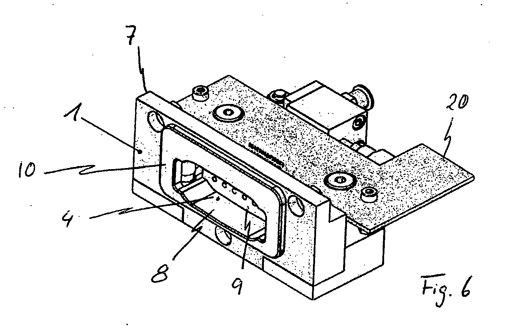

Gemäß der Figuren 5 und 6 besteht der erfindungsgemäße Stecker aus einem Steckergehäuse 21 vorzugsweise aus zwei miteinander verbundenen Kunststoffschalen, welche ein Einschubelement 22 mit integrierten Kontaktstiften 23 und Hydraulikanschlüssen 24 zumindest abschnittsweise umschließt. Das Einschubelement 22 hat vorliegend eine Anzahl von Schleifkontakten oder Kontaktstiften, welche hinsichtlich ihrer Form den steckdosenseitigen Kontakten für einen elektrischen Kontaktschluss angepasst sind. Auch die hydraulischen/pneumatischen Anschlüsse 24 sind an die steckdosenseitigen Anschlüsse hinsichtlich Position und Größe angepasst.

Demzufolge hat das Einschubelement 22 des erfindungsgemäßen Steckers 2 einen Mehrzahl von längs sich erstreckenden Durchgangsbohrungen, welche an der Vorderseite des Einschubelements 22 von ringförmigen Nuten umgeben werden, und die an der Rückseite des Einschubelements 22 mit Anschlusseinrichtungen vorzugsweise in Form von Anschlusszapfen 25 für das dichte Anschließen von Hydraulik/Pneumatikdruckschläuchen versehen sind. Im vorliegenden Ausführungsbeispiel ragen Teile dieser Anschlusszapfen 25 für die Hydraulikschläuche an der Ober-und/oder Unterseite des Steckergehäuses 21 aus diesem vor.

Des weiteren hat das Einschubelement 22 an seinem vorderen Abschnitt zumindest im seitlichen Bereich wulstförmige, zur Seite vorragende Vorsprünge 26, welche in Einschubrichtung gesehen beidseitige Hinterschneidungen definieren.

Schließlich ist das Einschubelement 22 in einer bevorzugten Ausführungsform (jedoch nicht unbedingt erforderlich) mit zwei Spreizlaschen 27 ausgebildet, die sich in Einschubrichtung von der Stirnseite des Einschubelements 22 aus erstrecken und somit eine L-förmige Verlängerung der seitlich vorstehenden wulstförmigen Vorsprünge 26 in Axialrichtung des Einschubelements 22 darstellen.

Bezüglich der Funktionsweise sowie der erzielbaren Vorteile der erfindungsgemäßen Einrichtung wird folgendes ausgeführt:

Für das gleichzeitige Herstellen einer elektrischen sowie hydraulischen/pneumatischen Verbindungen wird der erfindungsgemäße Stecker 2 gemäß vorstehender Beschreibung in den Einschubschacht 4 der erfindungsgemäßen Steckdose 1 eingeführt. Dabei kommen die Kontaktstifte oder Schleifkontakte des Steckers bei Überschreiten eines vorbestimmten Einführwegs mit den Kontaktklemmen oder Schleifkontakten der Steckdose 1 in elektrischen Kontakt miteinander.

Mit dem Einführen des steckerseitigen Einschubstücks 22 in den Einschubschacht 4 der Steckdose 1 gelangen ferner die beiden Spreizlaschen 27 (oder, falls nicht vorhanden, die seitlichen Vorsprünge 26) des Steckers 2 mit den steckdosenseitigen Klemmwalzen 14 in Eingriff und drücken diese entgegen deren Federvorspannungen aus den seitlichen Öffnungen 12 der den Einschubschacht 4 ausbildenden Buchse 8. Auf diese Weise kann das Einführen des steckerseitigen Einschubelements 22 in die steckdosenseitige Buchse 8 im Wesentlichen ohne größeren Widerstand durchgeführt werden.

Sobald die hydraulischen/pneumatischen Anschlussöffnungen an der Stirnseite des steckerseitigen Einschubelements 22 mit den Dichtringen auf Seiten der Steckdose 1 in Anlage kommen, positionieren sich gleichzeitig die beiden Klemmwalzen 14 an beiden Seiten des Einschubelements 22 unmittelbar hinter den seitlichen wulstförmigen Vorsprüngen 26. Hierdurch wird der Stecker 2 durch die auf die Klemmwalzen 14 einwirkenden Vorspannkräfte der beiden Vorspannfedern 15 mechanisch in dem Einschubschacht 4 der Steckdose 1 gehalten.

In einem bevorzugten Ausführungsbeispiel der Erfindung ist ein Hydraulik-/Pneumatikanschluss der Stecker-SteckdosenVerbindung mit dem hydraulischen/pneumatischen Betätigungsmechanismus 16 für die Hebel 13 fluidverbunden. Sobald nunmehr diese hydraulische/pneumatische Verbindung von einer Fluiddruckquelle (nicht gezeigt) druckbeaufschlagt wird, gelangt dieses Druckmedium über die Stecker-Steckdosenverbindung zu dem hydraulischen/pneumatischen Betätigungsmechanismus 16, wodurch die beiden Enden der Klemmhebel 13 auseinandergedrückt werden. Dabei werden die beiden Klemmwalzen 14 über den Klemmhebelmechanismus gegen das Gehäuse 21 des Steckers 2 sowie partiell in Einschubrichtung des Steckers 2 gegen die beiden seitlich vorragenden wulstförmigen Vorsprünge 26 gedrückt, wodurch die hydraulischen Anschlüsse dichtend aneinander gepresst werden.

Alternativ (nicht Teil der Erfindung) zu dieser Ausgestaltung kann es jedoch auch vorgesehen sein, dass in dem Augenblick, in welchem mit federelastischem Einschnappen der sich gegenüberliegenden Klemmwalzen 14 eine elektrische Verbindung zwischen einem stecker- sowie steckdosenseitigen Kontakt schließt, ein elektrisches Signal durch die steckdosenseitige Steuerplatine 20 an den Betätigungsmechanismus 16 abgegeben wird, worauf der Betätigungsmechanismus 16 beispielsweise in Form der vorstehend beschriebenen Kolben-/ZylinderAnordnung oder einer elektromotorischen oder Piezo-Antriebseinrichtung die Klemmwalzen 14 gegen das Steckergehäuse sowie die seitlich vorragenden Vorsprünge 26 verstemmt.

Konkreter beschrieben ist ein Sensor beispielsweise in Form einer sich elektrisch schließenden Drahtbrücke, einer optischen Lichtschranke, eines mechanisch betätigbaren Mikroschalters, eines magnetische betätigbaren Reed-Relais oder Hallsensors oder eines induktiven oder kapazitiven Schwingkreises angeordnet, durch den das Vorhandensein eines Steckers innerhalb der Steckdosenbuchse 8 detektierbar ist, woraus das von Sensor entsprechend abgegebene Signal zu einem Aktivierungssignal für den Betätigungsmechanismus 16 verarbeitet wird.

Schließlich kann es alternativ (und ggf. zusätzlich) auch vorgesehen sein, den Betätigungsmechanismus 16 manuell durch Anlegen eines entsprechenden elektrischen oder hydraulisch/pneumatischen Signals zu aktivieren, unabhängig vom Einführvorgang des Steckers 2 in die erfindungsgemäße Steckdose.

Durch die erfindungsgemäße Steckereinrichtung werden hydraulische/pneumatische sowie elektrische Verbindungen gleichzeitig mit Einstecken eines Steckers 2 in eine entsprechende Steckdose 1 hergestellt. Dabei sind die hydraulischen/pneumatischen Anschlüsse wie auch die elektrischen Anschlüsse in einem gemeinsamen steckerseitigen Einschubelement 22 integriert. Durch diese Ausgestaltung entfällt die Notwendigkeit der Anordnung von separaten elektrischen sowie hydraulischen/pneumatischen Anschlüssen, welche für deren Handhabung einen entsprechenden Mindestbauraum benötigen.

Des weiteren ist es erfindungsgemäß vorgesehen, die Steckdose 1 mit einer elektromotorischen oder pneumatischen/hydraulischen Verriegelungseinrichtung 16 auszubilden, welche vorzugsweise selbsttätig mit dem Einführen des Steckers 2 in die Steckdose betätigt wird und dabei zum Einen die hydraulischen/pneumatischen Anschlüsse fluiddicht verriegelt und zum Anderen ein unbeabsichtigtes Lösen bzw. Abziehen des Steckers 2 aus der Steckdose 1 verhindert.

Dabei hat es sich gezeigt, dass insbesondere die Anordnung von mechanischem, manuell betätigbaren Verriegelungseinrichtungen, wie sie aus dem Stand der Technik bekannt sind, gegen ein unbeabsichtigtes Lösen insbesondere dann, wenn die hydraulischen/pneumatischen Leitungen noch unter Druck stehen, nicht oder nur schwer gesichert werden können, wohingegen in der vorliegenden erfindungsgemäßen Ausführungsform die Verriegelung quasi selbsttätig mit Einführen des steckerseitigen Einschubelements 22 in den Aufnahmeschacht 4 der Steckdose 1 oder mit Druckbeaufschlagung zumindest eines der hydraulischen/pneumatischen Leitungen (nach dem Prinzip eines Überdruckventils) aktiviert wird.

Letztere Ausführungsform hat den Vorteil, dass mit Druckentspannung der betreffenden Druckleitung die Verriegelungseinrichtung 16 quasi selbsttätig gelöst wird, wohingegen jene Klemm- bzw. Betätigungseinrichtungen 16, welcher durch den Einführvorgang des Steckers 2 in die Steckdose 1 oder durch ein entsprechend manuell ausgelöstes elektrisches oder hydraulisches/pneumatisches Signal aktiviert werden, erst durch ein entsprechendes elektrisches oder hydraulisches/pneumatisches Gegensignal manuell wieder gelöst werden können.

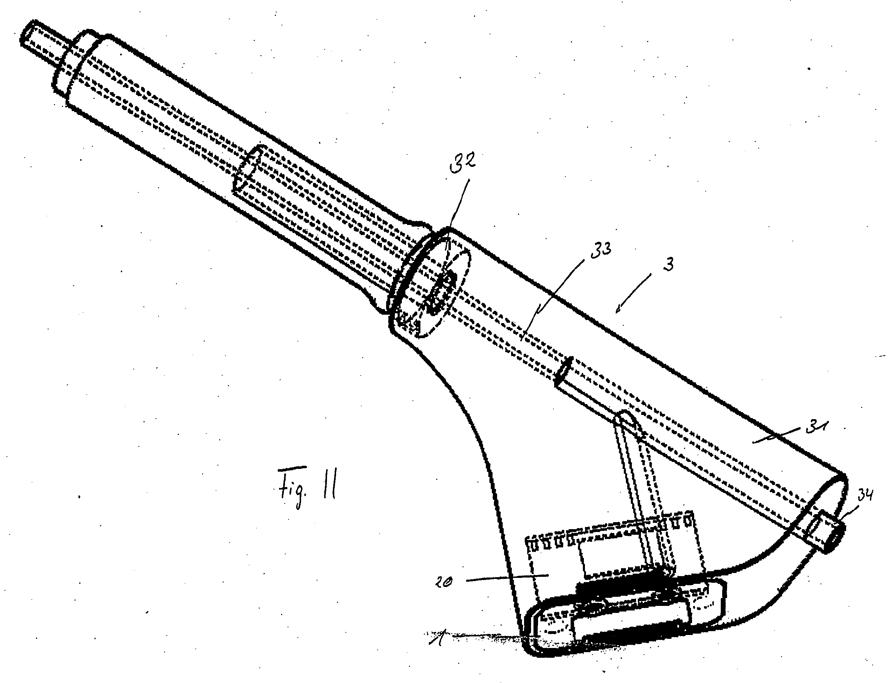

In der Fig. 11 wird ein bevorzugtes Anwendungsbeispiel für die erfindungsgemäße Steckereinrichtung gezeigt.

Aus dem Stand der Technik, insbesondere auf dem Gebiet der Medizintechnik ist es bekannt, an das proximale Ende von Endoskopen, Trokaren, Kathetern und dergleichen minimal invasiven Arbeitsgerätschaften so genannte Y-Stücke 3 anzudocken, über die zum Einen medizinische Geräte am distalen Ende des Endoskops/Trokars betätigt werden können, zum Anderen medizinische Wirkstoffe, Fluide oder weitere medizinische Arbeitsgerätschaften an das distale Ende des Endoskops/Trokars herangeführt werden können.

Moderne Gerätschaften dieser Gattung weisen dabei bewegliche distale Endstücke auf, welche elektrisch und/oder hydraulisch/pneumatisch betätigt werden müssen. Zu diesem Zweck sind diese gattungsgemäßen Gerätschaften an so genannten Versorgungsstationen elektrisch und/oder hydraulisch/pneumatisch angeschlossen, welche mit Betätigungseinrichtungen bspw. in Form von Steuerpulten ausgerüstet sind.

Da die Hygieneanforderungen ständig zunehmen und gleichzeitig aufwendige Reinigungsverfahren immer teuerer werden, steigt der Bedarf an so genannten Einwegvorrichtungen ständig. Daher ist es vorgesehen, Arbeitsgerätschaften der vorstehend genannten Gattung als ein solches Einwegprodukt zu gestalten, welches über eine ansteckbare Versorgungsleitung mit der Steuer- und Versorgungsstation verbindbar ist.

Daher weist ein allgemein aus dem Stand der Technik bekanntes Y-Stück 3 eines Endoskops/Trokars/Katheters oder dergleichen medizinische Arbeitsgerätschaft die erfindungsgemäße Steckdose 1 gemäß der Figuren 1 bis 6 auf, welche in das Gehäuse des Y-Stücks 3 integriert ist.

Demzufolge besteht das Y-Stück 3 aus einem vorzugsweise aus Kunststoff gefertigten Gehäuse 31 mit einem vorderen Anschlussstutzen 32, an den ein Endoskopschaft, Trokar oder Katheterschaft anschließbar ist. Dieser Anschlussstutzen weist eine innere Durchgangsbohrung 33 auf, die sich bis zu einer ersten hinteren Kanalöffnung 34 des Y-Stücks 3 erstreckt. Die erfindungsgemäße Steckdose 1 ist dabei unterhalb des im Y-Stück 3 ausgebildeten Durchgangskanals 33 angeordnet, wobei eine Anzahl von hydraulischen/pneumatischen Leitungen sowie elektrischen Verbindungen von der Steckdose ausgehend in den Y-stückseitigen Durchgangskanal 33 einmünden.

Alle wesentlichen, für die Betätigung der Arbeitsgerätschaft erforderlichen Leitungen erstrecken sich durch den Schaft in das Y-Stück 3 und enden dort an den elektrischen sowie hydraulischen/pneumatischen Anschlüssen der erfindungsgemäßen Steckdose 1. Sofern es sich bei der vorliegenden medizinischen Arbeitsgerätschaft um ein Einwegprodukt handelt, kann dieses für das Inbetriebnehmen mit der Steuerungs- und Versorgungsstation (nicht gezeigt) über die erfindungsgemäße Steckereinrichtung elektrisch sowie fluidtechnisch verbunden werden sowie nach dessen Gebrauch durch Abziehen des erfindungsgemäßen Steckers 2 aus der erfindungsgemäß im Y-Stück untergebrachten Steckdose 1 von der Steuerungs- und Versorgungsstation wieder getrennt werden, ohne dass diese von der Arbeitsgerätschaft kontaminiert wird. Wenn auch auf Seiten der nicht weiter dargestellten Steuer- und Versorgungsstation ebenfalls eine erfindungsgemäße Steckdose 1 vorgesehen ist, lässt sich auch das zur Betätigung und Versorgung notwendige Schlauchpaket, welches demzufolge an beiden Enden einen erfindungsgemäßen Stecker aufweist, von der Steuerungs- und Versorgungsstation abtrennen, um einem Reinigungs- und/oder Desinfektionsvorgang unterzogen zu werden.

## Patentansprüche

1. Kombinationssteckdose zur gleichzeitigen Herstellung von elektrischen sowie hydraulischen/pneumatischen Verbindungen mit einem vorzugsweise schachtförmigen Aufnahmeelement (8), in welchem elektrische Kontakte (5) sowie hydraulische/pneumatische Anschlüsse (6) integriert sind sowie einem hydraulisch betätigbaren Halte-Nerriegelungsmechanismus (13, 14, 15, 16) für ein Halten und Verriegeln des Kontaktschlusses mit einem Stecker, **dadurch gekennzeichnet, dass** der Halte-/Verriegelungsmechanismus (13, 14, 15, 16) zumindest einen schwenkbar oder linear bewegbar in einem Steckdosengehäuse gelagerten Hebel (13) aufweist, an dessen einem Ende eine Betätigungseinrichtung (16) für ein Verschwenken oder Verschieben des Hebels (13) angelenkt ist und an dessen anderem Ende ein Andrückelement in Form einer Klemmwalze (14) für ein in Anlage kommen mit dem eingeschobenen Stecker vorgesehen ist, wobei der Hebel (13) durch eine Vorspannfeder (15) kraftbeaufschlagt ist, um eine Haltekraft auf den eingeschobenen Stecker auszuüben und dass
die Betätigungseinrichtung (16) durch den Einführvorgang des Steckers in die Steckdose (1) für ein Verriegeln aktiviert wird, indem die Aktivierung der Betätigungseinrichtung (16) ausgelöst wird durch Druckbeaufschlagung zumindest eines der hydraulischen/pneumatischen Anschlüsse (6) der Steckdose (1) über den Stecker.

2. Kombinationsstecker (2) für eine Kombinationssteckdose gemäß Anspruch 1 zur gleichzeitigen Herstellung von elektrischen sowie hydraulischen/pneumatischen Verbindungen mit einem Einschubelement (22), in welchem eine Anzahl von elektrischen sowie hydraulischen/pneumatischen Anschlüssen (23, 24) integriert sind,
**gekennzeichnet durch**
eine Anzahl von, in Einführrichtung wirkende Hinterschneidungen ausbildende, wulstförmige Vorsprünge, die in Einschubrichtung gesehen am vorderen Abschnitt im beidseitigen Bereich des Einschubelements (22) ausgebildet sind zur Aufnahme von in Einschubrichtung des Stecker (2) wirkenden Halte-/Verriegelungskräften eines steckdosenseitigen Halte-/Verriegelungsmechanismus (13, 14, 15, 16).

## Claims

1. A combined socket for simultaneously establishing electric as well as hydraulic/pneumatic connections comprising a preferably slot-like receiving element (8) in which electric contacts (5) and hydraulic/pneumatic ports (6) are integrated as well as a retaining and locking mechanism (13, 14, 15, 16) for retaining and locking the positive contact with a plug that can be hydraulically actuated, **characterized in that**
the retaining and locking mechanism (13, 14, 15, 16) has at least one lever (13) supported in a socket casing in a pivotable or linearly movable manner, at the one end of which an actuating means (16) is hinged for pivoting or displacing the lever (13) and at the other end of which a clamping roller shaped pressing member (14) is provided for coming into contact with the inserted socket, wherein the lever (13) is biased by a bias spring (15) to apply a retaining force to the inserted socket; and
the actuating means (16) is activated for locking by the inserting operation of the plug into the socket (1) while activation of the actuating means (16) is triggered by applying pressure to at least one of the hydraulic/pneumatic ports (6) of the socket (1) via the plug.

2. A combined plug (2) for a combined socket according to claim 1, for simultaneously establishing electric as well as hydraulic/pneumatic connections, comprising a plug-in element (22) in which a number of electric and hydraulic/pneumatic terminals are integrated, **characterized by**
a number of undercuts forming and bead-shaped projections acting in the inserting direction, which are configured at a front section in a bilateral area of the plug-in elements (22) with respect to the inserting direction, for receiving retaining and locking forces of a socket-side locking mechanism (13, 14, 15, 16) acting in the inserting direction of the plug (2).

## Revendications

1. Prise à combinaison pour l'établissement simultané de liaisons électriques et hydrauliques/pneumatiques comprenant un élément de réception (8) de préférence en forme de gaine, dans lequel sont intégrés des contacts (5) électriques et des branchements (6) hydrauliques/pneumatiques et un mécanisme de retenue/verrouillage (13, 14, 15, 16) pouvant être actionné de façon hydraulique pour une retenue et un verrouillage de la fermeture de contact avec une fiche, **caractérisée en ce que** le mécanisme de retenue/verrouillage (13, 14, 15, 16) présente au moins un levier (13) fixé dans un boîtier de prise de façon basculante ou mobile dans le sens linéaire, levier sur une extrémité duquel est articulé un dispositif d'actionnement (16) pour un basculement ou un déplacement du levier (13) et sur l'autre extrémité duquel est prévu un élément d'appui sous la forme d'un cylindre de serrage (14) pour une application avec la fiche introduite, le levier (13) étant sollicité en force par un ressort de pré-tension (15) pour exercer une force de retenue sur la fiche introduite et **en ce que**
le dispositif d'actionnement (16) est activé par la procédure d'introduction de la fiche dans la prise (1) pour un verrouillage par le fait que l'actionnement du dispositif d'actionnement (16) est déclenché par sollicitation en pression d'au moins l'un des branchements (6) hydrauliques/pneumatiques de la prise (1) au moyen de la fiche.

2. Fiche à combinaison (2) pour une prise à combinaison selon la revendication 1 pour l'établissement simultané de liaisons électriques et hydrauliques/pneumatiques comprenant un élément d'introduction (22), dans lequel un certain nombre de branchements (23, 24) électriques et hydrauliques/pneumatiques sont intégrés,
**caractérisée par**
un certain nombre de saillies en forme de renflement, formant des contre-dépouilles agissant dans le sens d'introduction, qui, vues dans le sens d'introduction, sont conçues sur la partie avant dans la zone des deux côtés de l'élément d'introduction (22) pour la réception de forces de retenue/verrouillage, agissant dans le sens d'introduction de la fiche (2), d'un mécanisme de retenue/verrouillage (13, 14, 15, 16) côté prise.
